# DEMANDE DE BREVET EUROPEEN

(11) **EP 4 813 429 A1**
(43) Date de publication de la demande: **30.09.2026**
(21) Numéro de dépôt: 26154275.7
(22) Date de dépôt: 27.01.2026
(51) Int. Cl.: A61M 16/00, A61M 16/10, A61M 16/12

(54) **VENTILATEUR MÉDICAL À ATTÉNUATEURS ACOUSTIQUES MULTIFRÉQUENCE**

(30) Priorité: 27.03.2025 FR 2503147
(71) Demandeur: Air Liquide Medical Systems, 92182 Antony Cedex (FR)
(72) Inventeur: LOPEZ, Julien, 92182 Antony Cedex (FR); ROUSSILLOT, Mathieu, 92182 Antony Cedex (FR)
(74) Mandataire: Air Liquide

(57) **Abrégé**

L'invention concerne un ventilateur médical (1) comprenant : un caisson à turbine (10) comprenant un compartiment interne (11) logeant une turbine motorisée (12), et un circuit d'air (15) reliant fluidiquement une entrée d'air (13) au compartiment à turbine (11), et des moyens de pilotage pilotant la turbine motorisée (12). Des chambres d'atténuation sonore (20, 30, 40) permettent de stopper les ondes sonores générées par le flux d'air circulant, entre 400 et 2000 Hz afin de rendre le ventilateur (1) plus silencieux et ce, sans nécessiter une utilisation de mousses isolantes.

## Description

L'invention concerne un ventilateur médical équipé d'un caisson interne, de préférence extractible, comprenant un circuit d'air, une entrée d'oxygène et une turbine motorisée, et des atténuateurs acoustiques multifréquence en communication fluidique avec le circuit d'air, permettant d'atténuer le bruit, i.e. ondes sonores, généré par l'aspiration et/ou la circulation d'air au sein du caisson.

Un ventilateur médical est un appareil d'assistance respiratoire servant à délivrer une aide respiratoire, c'est-à-dire une ventilation artificielle, à une personne, à savoir un patient souffrant de troubles ou insuffisances respiratoires, plus ou moins sévères, pouvant résulter de différentes pathologies ou analogues.

Pendant son fonctionnement, le ventilateur médical aspire de l'air ambiant, le filtre et le délivre ensuite au patient sous forme d'air filtré pouvant être enrichi en oxygène, et ce, au moyen d'une turbine motorisée agencée dans la carcasse périphérique du ventilateur. Habituellement, la turbine motorisée, aussi appelée (micro)soufflante, pompe ou compresseur, comprend un moteur électrique entrainant une roue rotative agencée dans une volute. Elle est alimentée en air ambiant par un circuit d'admission d'air qui comprend le filtre. L'air circule dans le circuit d'admission d'air en étant aspiré par la roue pendant ses rotations lorsqu'elle est entrainée par le moteur électrique, lorsqu'il est commandé pour fonctionner.

L'utilisation d'une turbine motorisée aspirant l'air via le circuit interne d'admission d'air engendre un problème de bruit lié à la circulation de l'air dans le conduit d'admission d'air, qui peut être très perturbant ou gênant pour les utilisateurs, typiquement le personnel médical, ou les patients recevant l'air délivré par la turbine du ventilateur, en particulier pendant la nuit où un calme relatif règne dans les hôpitaux. Une grande partie du bruit résulte de l'écoulement de l'air dans le conduit d'admission d'air qui est relié à l'atmosphère.

Une solution connue repose sur l'utilisation de mousses acoustiques servant à absorber ou atténuer les nuisances sonores. De telles mousses étaient couramment utilisées dans les ventilateurs médicaux équipés de caissons dit « à turbine » extractibles présentant un volume interne important, typiquement des caissons de forme sensiblement parallélépipédique.

Toutefois, utiliser de telles mousses n'est idéale, ni souhaitable car peuvent engendrer des problèmes de performances ventilatoires, de libération intempestives de particules du fait de leur attrition/usure au fil du temps...

Afin de supprimer ces mousses, FR3146599 a proposé d'agencer, dans le circuit ou conduit d'admission d'air, deux résonateurs différents, à savoir un résonateur quart-d'onde et un résonateur d'Helmoltz, de sorte de réduire le niveau sonore grâce à un « piégeage » des ondes sonores au sein de ces résonateurs.

Si cette solution est efficace lorsque le circuit ou conduit d'admission d'air est sensiblement rectiligne, donc d'architecture simple, elle n'est pas adaptée aux ventilateurs médicaux équipés d'un boitier ou caisson interne, appelé « caisson à turbine », comprenant une entrée et une sortie de gaz traversant la paroi du caisson, une turbine motorisée agencée dans un compartiment à turbine et une chambre interne de volume important mettant en liaison fluidique l'entrée de gaz et le compartiment à turbine afin de permettre une circulation du gaz, tel de l'air, depuis l'entrée de gaz du caisson vers l'aspiration de la turbine, comme décrit par EP2707603.

D'autres solutions ont été proposées par CN117018363, US2012/121441, US2023/310772, CN117404338 et US2021/001069. Toutefois, elles trouvent toutes des limitations, notamment de ne pas pouvoir éliminer efficacement les ondes sonores dans des plages de fréquences larges.

Un problème est de pouvoir éliminer ou diminuer les nuisances sonores, c'est-à-dire le bruit, généré par l'écoulement de l'air dans le caisson à turbine agencé dans un ventilateur médical, pendant le fonctionnement de la turbine, de manière à limiter la gêne sonore pour le personnel soignant et les patients, donc de proposer un ventilateur médical plus silencieux, lequel ne requiert pas d'utilisation de mousses d'insonorisation, en particulier qui conduise à une élimination d'ondes sonores de différentes fréquences.

Une solution de l'invention concerne alors un ventilateur médical, c'est-à-dire un appareil d'assistance respiratoire, comprenant : un caisson à turbine (i.e. boitier) comprenant un compartiment interne logeant une turbine motorisée, et un circuit d'air reliant fluidiquement une entrée d'air au compartiment à turbine et des moyens de pilotage pilotant, i.e. contrôlant, (au moins) la turbine motorisée.

De plus, le ventilateur médical selon l'invention comprend :
- une première chambre d'atténuation sonore et un premier canal de liaison dimensionnés et conformés pour former un premier résonateur de Helmholtz configuré pour piéger, stopper et/ou atténuer des ondes sonores de fréquence comprise entre 500 et 1000 Hz,
- une deuxième chambre d'atténuation sonore et un deuxième canal de liaison dimensionnés et conformés pour former un deuxième résonateur de Helmholtz configuré pour piéger, stopper et/ou atténuer des ondes sonores de fréquence comprise entre 400 et 900 Hz,
- une troisième chambre d'atténuation sonore et un troisième canal de liaison dimensionnés et conformés pour former un troisième résonateur de Helmholtz configuré pour piéger, stopper et/ou atténuer des ondes sonores de fréquence comprise entre 1000 et 2000 Hz, et
- au moins une quatrième chambre d'atténuation sonore, via au moins une plaque perforée, de préférence ayant un taux de perforation compris entre 1 et 2%, la quatrième chambre d'atténuation sonore et ladite au moins une plaque perforée étant configurées pour piéger, stopper et/ou atténuer des ondes sonores de fréquence comprise entre 800 et 1500 Hz.

Grâce à l'invention, on peut éliminer et/ou diminuer les nuisances sonores, c'est-à-dire le bruit, généré par l'écoulement de l'air dans le circuit d'air du caisson à turbine du ventilateur médical, pendant le fonctionnement de la turbine, dans des plages de fréquences larges, à savoir entre 400 et 2000 Hz, ce qui rend le ventilateur plus silencieux, donc engendrant une gêne sonore réduite pour le personnel soignant et les patients.

Selon le mode de réalisation considéré, le ventilateur médical selon l'invention peut comprendre l'une ou plusieurs des caractéristiques suivantes :
- les premier, deuxième et troisième canaux de liaison sont aussi appelés « cols ».
- les premier, deuxième et troisième canaux de liaison ont une forme allongée, de préférence rectiligne.
- les premier, deuxième et troisième canaux de liaison ont une longueur comprise entre 8 et 20 mm, de préférence entre 10 et 15 mm.
- les premier, deuxième et troisième canaux de liaison se projettent vers l'intérieur des première, deuxième et troisième chambres d'atténuation sonore.
- le circuit d'air est en communication fluidique avec la première chambre d'atténuation sonore via le premier canal de liaison ; la deuxième chambre d'atténuation sonore via le deuxième canal de liaison ; et la troisième chambre d'atténuation sonore via le troisième canal de liaison.
- le circuit d'air est relié fluidiquement aux première, deuxième et troisième chambres d'atténuation sonore par les premier, deuxième et troisième canaux de liaison.
- la première chambre d'atténuation sonore, la deuxième chambre d'atténuation sonore et la troisième chambre d'atténuation sonore sont configurées pour absorber et/ou atténuer les ondes sonores générées par et/ou résultant dans la circulation du flux d'air dans le circuit d'air.
- le circuit d'air est configuré pour acheminer de l'air entrant par l'entrée d'air jusqu'au compartiment à turbine.
- le caisson a une forme parallélépipédique rectangle ou une autre forme.
- le caisson comprend une entrée de gaz et une sortie de gaz traversant la paroi périphérique du boitier.
- les moyens de pilotage sont des moyens de pilotage électroniques.
- les moyens de pilotage comprennent au moins un microprocesseur.
- la turbine motorisée comprend un moteur électrique.
- le moteur électrique est alimenté électriquement par des câbles électriques ou analogues.
- le caisson comprend deux demi-coques solidarisées l'une à l'autre
- un joint d'étanchéité est agencé entre les deux demi-coques afin d'assurer une étanchéité gazeuse entre les deux demi-coques.
- les deux demi-coques sont complémentaires l'une de l'autre et configurées pour s'assembler l'une à l'autre, par exemple s'emboiter l'une dans l'autre, de préférence de manière étanche de sorte d'empêcher toute fuite gazeuse au niveau de leurs périmètres de jonction.
- les deux demi-coques sont rigides, de préférence en polymère, par exemple en ABS/PC.
- de préférence, le circuit d'air du caisson est tortueux, c'est-à-dire non rectiligne.

En particulier, il comprend une ou des portions rectilignes et/ou une ou des portions courbes, i.e. recourbées.
- alternativement, le circuit d'air du caisson peut être rectiligne ou sensiblement rectiligne.
- le circuit d'air comprend des passages de gaz délimités par des parois internes au caisson.
- le circuit d'air comprend une portion amont linéaire comprenant l'entrée de gaz, c'est-à-dire rectiligne ou approximativement rectiligne.
- le circuit d'air comprend une portion aval linéaire relié fluidiquement au compartiment interne ou débouchant dans le compartiment interne logeant la turbine, c'est-à-dire rectiligne ou approximativement rectiligne.
- le circuit d'air comprend une portion de jonction, au moins partiellement courbe, reliant fluidiquement lesdites portions amont et aval, de manière à assurer une circulation du flux d'air entre lesdites portions amont et aval via la portion de jonction courbe.
- le premier canal de liaison (i.e. premier col) est en liaison fluidique avec la portion de jonction du circuit d'air, c'est-à-dire qu'il vient se raccorder à la portion de jonction courbe.
- le troisième canal de liaison (i.e. troisième col) est en liaison fluidique avec la portion aval linéaire du circuit d'air, c'est-à-dire qu'il vient se raccorder à la portion aval linéaire.
- le deuxième canal de liaison (i.e. deuxième col) est en liaison fluidique avec la portion de jonction et/ou la portion aval linéaire du circuit d'air, en particulier le deuxième canal de liaison est relié à la jonction desdites portion de jonction et portion aval linéaire du circuit d'air, c'est-à-dire dans la région de raccordement desdites portion de jonction et portion aval linéaire.
- lesdites portions amont et aval sont agencées sensiblement parallèles l'une à l'autre, c'est-à-dire parallèles ou approximativement parallèles.
- selon un mode de réalisation, la portion aval linéaire du circuit d'air est relié fluidiquement au compartiment interne contenant la turbine par une portion intermédiaire aval, de préférence au moins partiellement une portion courbe.
- le premier, le deuxième et le troisième résonateur de Helmholtz sont configurés pour piéger, stopper et/ou atténuer des ondes sonores de fréquences égales ou différentes.
- avantageusement, la première chambre d'atténuation sonore a un volume différence, i.e. supérieur ou inférieur, à celui des deuxième et troisième chambres d'atténuation sonore.
- les chambres d'atténuation sonore ont un volume interne compris entre 10 et 90 cm³.
- les résonateurs sont configurés pour réduire ou atténuer le niveau sonore, c'est-à-dire le bruit généré par l'écoulement de l'air dans le circuit d'air, pendant le fonctionnement du ventilateur médical, i.e. le fonctionnement de la turbine avec aspiration d'air du fait des rotations de la roue à ailette de la turbine, et ainsi limiter la gêne sonore pour le personnel soignant et les patients, en particulier en milieu hospitalier.
- le circuit d'air est en communication fluidique avec ladite au moins une quatrième chambre d'atténuation sonore via ladite au moins une plaque perforée.
- ladite au moins une quatrième chambre d'atténuation sonore est agencée en aval des première et deuxième chambres d'atténuation sonore, en considérant le sens de circulation de l'air dans le circuit d'air.
- la quatrième chambre d'atténuation sonore est en communication fluidique avec la portion aval linéaire du circuit d'air.
- la quatrième chambre d'atténuation sonore a un volume interne compris entre 10 et 45 cm³, de préférence entre 15 et 32 cm³.
- la quatrième chambre d'atténuation sonore est sous-divisée en plusieurs compartiments d'atténuation sonore.
- la quatrième chambre d'atténuation sonore est sous-divisée en deux compartiments d'atténuation sonore séparés par une paroi intercalaire.
- la quatrième chambre d'atténuation sonore est fermée par une ou plusieurs plaques perforées, de préférence par deux plaques perforées juxtaposées.
- la ou chaque plaque perforée a une épaisseur comprise entre 0,1 et 2 mm, de préférence entre 0,5 et 1 mm.
- la ou chaque plaque perforée est formé d'un matériau de type polymère, de préférence de type ABS/PC.
- la ou chaque plaque perforée a un taux de perforation compris entre 1 et 2%, de préférence moins de 1,5% (i.e. aire perforée/aire totale).
- la ou chaque plaque perforée est traversée par des trous formant un quadrillage.
- la ou chaque plaque perforée est traversée par des trous ayant un diamètre compris entre 0,3 et 0,8 mm.
- la ou chaque plaque perforée est extractible, c'est-à-dire amovible de manière à pouvoir être extraite.
- la ou chaque plaque perforée a une épaisseur inférieure à 10 mm, de préférence entre 0,4 et 0,8 mm, par exemple de l'ordre de 0,6 mm.
- au moins le compartiment interne logeant la turbine, le circuit d'air, les chambres d'atténuation sonore et les canaux de liaison sont délimités par des parois internes agencées dans le caisson.
- le compartiment interne logeant la turbine est au moins partiellement délimité par des parois conformées pour épouser les contours de la turbine, en particulier les contours de la volute et du carter contenant le moteur, c'est-à-dire que les parois délimitant le compartiment interne comprennent une empreinte de la forme extérieure de la turbine.
- selon un mode de réalisation, les parois internes sont formées d'une pièce avec l'une ou des parois périphériques du caisson, par exemple par moulage par injection.
- selon un autre mode de réalisation, au moins une partie des parois internes sont agencées sur une ou des structures amovibles, i.e. extractibles, venant se loger dans le caisson.

Par ailleurs, selon le mode de réalisation considéré, le ventilateur médical selon l'invention peut comprendre aussi l'une ou plusieurs des caractéristiques additionnelles suivantes :
- la turbine motorisée comprend un moteur électrique comprenant un axe-moteur et surmonté par une volute.
- la volute comprend une entrée de volute, une sortie de volute, en particulier un conduit de sortie, et un compartiment à roue logeant une roue à ailettes portée par l'axe-moteur et mobile en rotation lors des rotations dudit axe-moteur.
- la turbine motorisée est une turbine, une (micro)soufflante, une pompe, un compresseur ou analogue.
- l'entrée de volute et la sortie de volute sont en communication fluidique avec le compartiment à roue de la volute de sorte que l'air puisse entrer dans le compartiment à roue par l'entrée de volute et ressortir dudit compartiment à roue par la sortie de volute, en particulier par le conduit de sortie de volute.
- l'entrée d'air du circuit d'air est reliée fluidiquement à l'atmosphère.
- la sortie de volute, typiquement le conduit de sortie de volute, est reliée fluidiquement, via une sortie d'air du ventilateur, à un circuit patient, tel un tuyau flexible, venant se raccorder à ladite sortie d'air du ventilateur.
- le circuit patient est raccordé fluidiquement à une interface respiratoire, tel un masque ou analogue, fournissant le gaz respiratoire au patient, tel de l'air ou un mélange air/O₂.
- des moyens de pilotage, c'est-à-dire un dispositif ou une unité de pilotage, contrôlent le fonctionnement du moteur de la turbine, donc les rotations, de la roue à ailettes.
- la volute définit un compartiment interne contenant la roue à ailettes qui est mobile en rotation, pendant ses rotations, c'est-à-dire lorsqu'elle est entrainée en rotation par le moteur électrique.
- le moteur électrique est un moteur du type sans balai.
- les rotations de la roue à ailettes engendrent une aspiration d'air via l'entrée de volute.
- au moins un élément de filtration d'air est agencé en amont de l'entrée d'air alimentant le circuit d'air entre l'entrée d'air.
- la turbine est contrôlé par les moyens de pilotage de manière à maintenir, pendant son fonctionnement, un régime constant, c'est-à-dire une vitesse de rotation constante ou, alternativement, des régimes différents, c'est-à-dire deux (ou plus) vitesses de rotation prédéfinies différentes, i.e. l'une étant supérieure à l'autre.
- le ventilateur comprend une carcasse extérieure forme une coque protectrice du ventilateur.
- le caisson à turbine est agencé et est maintenu dans la carcasse ou coque extérieure du ventilateur.
- le ventilateur comprend une poignée de portage afin de faciliter son transport par un utilisateur, en particulier la poignée est solidaire de la carcasse..
- les moyens de pilotage comprennent au moins une carte électronique.
- la carte électronique comprend un ou plusieurs microprocesseurs.
- le (ou les) microprocesseur met en oeuvre un ou des algorithmes assurant le fonctionnement du ventilateur, notamment le pilotage de la turbine, les affichages sur l'écran, le déclenchement des alarmes etc...
- la turbine, i.e. soufflante, pompe ou compresseur, fournit un gaz respiratoire, tel de l'air ou de l'air enrichi en oxygène.
- le ventilateur comprend un écran d'affichage, de préférence à affichage en couleurs.
- l'écran d'affichage permet d'afficher des informations, des courbes, des touches de sélection ou de réglage, des icônes ou autres.
- l'écran d'affichage fait partie d'une interface homme-machine (IHM)
- l'IHM comprend en outre un ou plusieurs boutons ou touches de réglage ou sélection actionnable par l'utilisateur.
- l'écran d'affichage est tactile, de préférence il comprend une ou des touches tactiles, i.e. des touches virtuelles, s'affichant sur l'écran d'affichage.
- la ou les touches tactiles permettant d'opérer des sélections, des validations, des réglages, des démarrages ou des arrêts de fonctionnement... Les touches tactiles sont à actionnement digital, c'est-à-dire qu'elles sont activées lorsque l'utilisateur appuie dessus avec son doigt, typiquement son index.
- l'écran d'affichage est configuré pour opérer un affichage d'informations sous forme de caractères alphanumériques, de représentations graphiques (e.g. graphes, courbes, dessins, icones, etc....), de photos, d'animations vidéo......ou autres.
- il comprend en outre des moyens d'alimentation en courant électrique.
- les moyens d'alimentation en courant électrique sont raccordés électriquement (directement ou indirectement) à la (aux) carte électronique, à l'écran d'affichage, à la turbine et plus généralement à tous les éléments ou composants du ventilateur requérant du courant électrique pour fonctionner.
- les moyens d'alimentation en courant électrique comprennent une batterie rechargeable.
- les moyens d'alimentation en courant électrique comprennent un ou des câbles électriques, en particulier des moyens de raccordement au secteur (110/220 V), tels que prise électrique, câbles, convertisseur de courant.
- les moyens de pilotage sont configurés pour commander la turbine de manière à délivrer du gaz au moins pendant les phases inspiratoires du patient.
- les moyens de pilotage sont configurés pour piloter les accélérations et les freinages et/ou les ralentissements du moteur électrique.
- la turbine est commandée pour atteindre une vitesse de rotation maximale d'au moins 30 000 tr/min, de préférence au moins 40 000 à 60 000 tr/min, voire même jusqu'à 70 000 tr/min.

Par ailleurs, l'invention porte sur un ventilateur médical comprenant un caisson à turbine comprenant un compartiment interne logeant une turbine motorisée, et un circuit d'air reliant fluidiquement une entrée d'air au compartiment à turbine, et des moyens de pilotage pilotant la turbine motorisée, dans lequel le circuit d'air est en communication fluidique avec : une première chambre d'atténuation sonore via un premier canal de liaison, une deuxième chambre d'atténuation sonore via un deuxième canal de liaison, et une troisième chambre d'atténuation sonore via un troisième canal de liaison, dans lequel :
- la première chambre d'atténuation sonore et le premier canal de liaison sont dimensionnés et conformés pour constituer un premier résonateur de Helmholtz,
- la deuxième chambre d'atténuation sonore et le deuxième canal de liaison sont dimensionnés et conformés pour constituer un deuxième résonateur de Helmholtz, et
- la troisième chambre d'atténuation sonore et le troisième canal de liaison sont dimensionnés et conformés pour constituer un troisième résonateur de Helmholtz
- et avantageusement, une quatrième chambre d'atténuation sonore dimensionnée et conformée pour constituer un quatrième résonateur de Helmholtz.

De préférence :
- les canaux de liaison ont une section inférieure à 70 mm² et une longueur inférieure à 15 mm,
- et/ou le circuit d'air comprend une portion amont linéaire comprenant l'entrée de gaz, une portion aval linéaire débouchant dans le compartiment interne logeant la turbine, et une portion de jonction reliant fluidiquement lesdites portions amont et aval, de préférence une portion de jonction au moins partiellement courbe,
- et/ou le premier canal de liaison est en liaison fluidique avec la portion de jonction du circuit d'air, le troisième canal de liaison est en liaison fluidique avec la portion aval linéaire du circuit d'air, et le deuxième canal de liaison est en liaison fluidique avec la portion de jonction et/ou la portion aval linéaire du circuit d'air, en particulier le deuxième canal de liaison est relié à la jonction desdites portion de jonction et portion aval linéaire du circuit d'air,
- et/ou ladite au moins une quatrième chambre d'atténuation sonore est en liaison fluidique avec la portion aval linéaire du circuit d'air et/ou séparée dudit circuit d'air par au moins une plaque perforée.

L'invention va maintenant être mieux comprise grâce à la description détaillée suivante, faite à titre illustratif mais non limitatif, en référence aux figures annexées parmi lesquelles :
Fig. 1 représente un mode de réalisation d'un ventilateur médical selon l'invention, montré ouvert.
Fig. 2 représente de l'architecture intérieure du caisson à turbine (i.e. l'une des demi-coques) équipant le ventilateur médical de Fig. 1, avec turbine agencée dans le compartiment à turbine.
Fig. 3 est analogue à Fig. 2, à l'exception de la turbine (non montrée) qui en a été extraite.
Fig. 4 est analogue à Fig. 2 et Fig. 3, et montre la turbine extraite et ses composants.
Fig. 5 schématise une partie du circuit d'air du caisson à turbine des Fig. 2 à Fig. 4, et les résonateurs de Helmholtz qui y sont reliés.

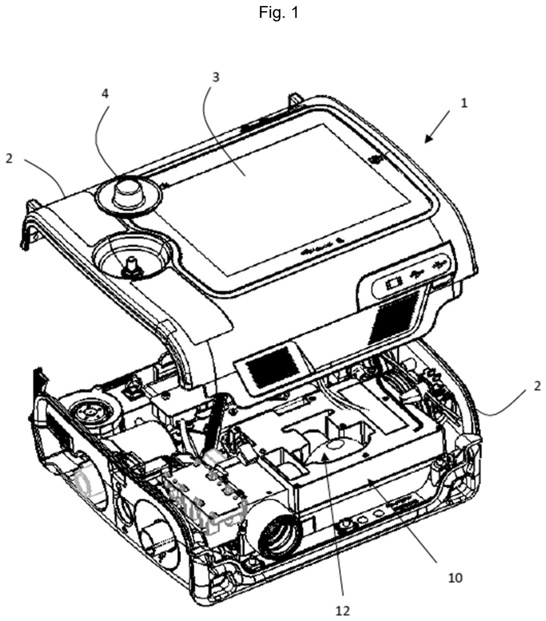

Fig. 1 représente un mode de réalisation d'un ventilateur médical 1, c'est-à-dire un appareil d'assistance respiratoire, délivrant une ventilation assistée à un patient selon l'invention, lequel est montré ouvert de manière à permettre de visualiser le caisson 10 extractible qui s'y trouve, comme expliqué ci-après.

Le ventilateur 1 est conçu pour fournir un gaz respiratoire à un patient, c'est-à-dire un gaz médical, en particulier de l'air provenant de l'atmosphère ambiante, éventuellement enrichi en oxygène, c'est-à-dire un mélange air/oxygène.

Le ventilateur médical 1 comprend classiquement une carcasse 2 rigide, par exemple en polymère, formée ici de deux parties ou demi-carcasses venant s'associer l'une à l'autre (i.e. une demi- carcasse supérieure et une demi- carcasse inférieure), dans laquelle sont agencés les différents éléments permettant son fonctionnement, en particulier une turbine 12 motorisée, aussi appelée pompe, (micro)soufflante, compresseur ou analogue, pour délivrer un flux de gaz respiratoire, e.g. air ou mélange air/O₂ ; des passages de gaz et/ou des éléments de gestion des flux, tels que vannes, clapets anti-retour... pour véhiculer le ou les flux gazeux ; des moyens de pilotage à microprocesseur(s), c'est-à-dire un dispositif ou une unité de pilotage ; des moyens d'alimentation électrique, par exemple une (ou des) batterie rechargeable, des câbles électriques... ; et des éléments d'alimentation en oxygène pour amener de l'oxygène et réaliser des mélanges air/O₂ ...

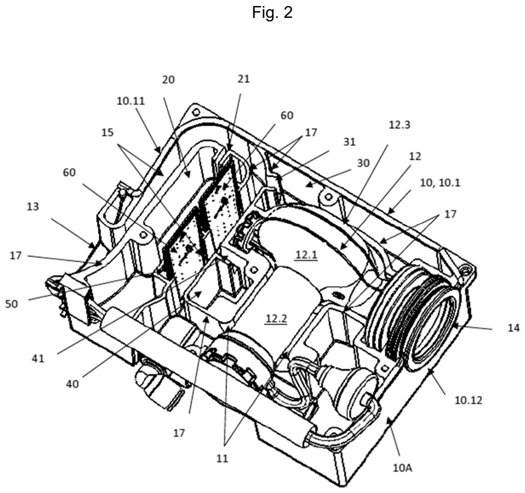

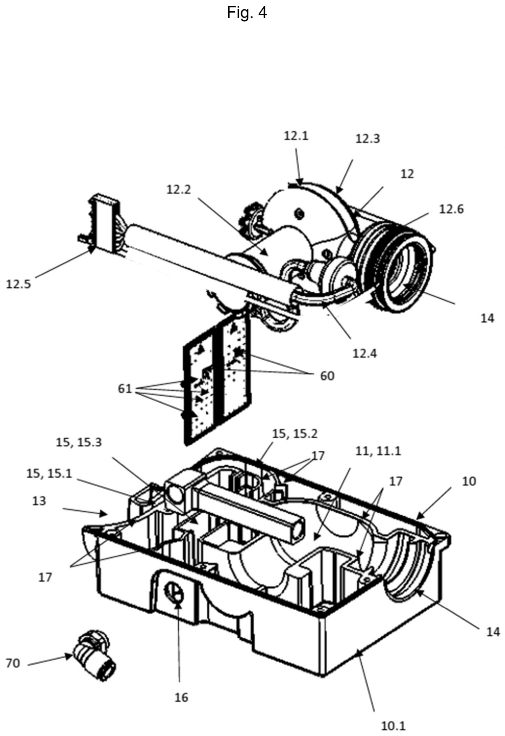

Le ventilateur médical 1 de l'invention est d'un type particulier étant donné que sa turbine 12 est agencée au sein d'un caisson ou boitier 10 de forme parallélépipédique rectangle, comme illustré sur Fig. 2 à Fig. 4. Le caisson 10 peut être en polymère type ABS/PC. Avantageusement, le caisson 10 est extractible ou amovible, c'est-à-dire qu'il peut être retiré de la carcasse du ventilateur médical 1.

Le caisson 10 comprend une entrée de gaz 13 par laquelle de l'air peut pénétrer dans le caisson 10, comme expliqué ci-après, et une sortie de gaz 14 par laquelle de l'air, éventuellement enrichi en oxygène, peut ressortir du caisson 10. Les entrée et sortie de gaz 13, 14 sont aménagées dans et/ou portées par des faces opposées 10.11, 10.12 de la paroi périphérique 10.1.

Avantageusement, le caisson 10 comprend deux demi-coques 10A, 10B solidarisées l'une à l'autre, par exemple par emboitement ou autre.

Ici, le caisson 10 est de forme parallélépipédique et ses dimensions sont de l'ordre de 15 à 20 cm en longueur, de 10 à 15 cm en largeur, et de 8 à 10 cm en hauteur. Toutefois, il pourrait avoir une autre forme et/ou d'autres dimensions.

Les deux demi-coques 10A, 10B sont assemblées le long d'un périmètre de jonction 18 comprenant préférentiellement un joint d'étanchéité 18.1 est agencé entre les deux demi-coques 10A, 10B. Les deux demi-coques 10A, 10B sont maintenues assemblées l'une de l'autre par des vis ou analogues (non montrées) venant se loger dans des perçages/logements à vis 19 ou analogues répartis autour du périmètre de jonction 18.

Comme visible sur Fig. 2 à Fig. 4, le caisson 10, en particulier les deux demi-coques 10A, 10B, comprend des parois internes 17 délimitant des passages, des volumes internes etc... comme détaillé ci-après. Ces parois 17 peuvent être réalisées en un matériau rigide de type polymère, i.e. plastique, par exemple ABS/PC.

En particulier, les parois 17 délimitent un circuit d'air 15, des chambres d'atténuation sonore 20-50, des canaux de liaison 21-41 et un compartiment à turbine 11 dans lequel est logée la turbine motorisée 12.

Comme on le voit, le compartiment à turbine 11 est conformé, à savoir sa paroi interne 17, pour présenter une empreinte tridimensionnelle 11.1 des contours externes de la turbine 12, en particulier de sa volute 12.1 et de son carter-moteur 12.2, c'est-à-dire que la paroi interne 17 du compartiment à turbine 11 est configuré pour épouser les contours externes de la turbine 12, tout en laissant un espacement entre eux afin de permettre une circulation du gaz provenant du circuit d'air 15 vers l'entrée 12.3 situé au-dessus de la volute 12.1. Chaque demi-coque 10A, 10B comprend donc une demi-empreinte tridimensionnelle 11.1 comprenant une demi-volute 12.1 et un demi-carter 12.2.

Par ailleurs, le circuit d'air 15 forme un passage pour l'air aspiré par la turbine 12, qui relie fluidiquement l'entrée d'air 13 au compartiment à turbine 11 logeant la turbine 12.

Plus précisément, selon l'invention le circuit d'air 15 est en communication fluidique avec plusieurs chambres d'atténuation sonore, à savoir une première chambre d'atténuation sonore 20 reliée au circuit d'air 15 par un premier canal de liaison 21, une deuxième chambre d'atténuation sonore 30 reliée au circuit d'air 15 par un deuxième canal de liaison 31, et une troisième chambre d'atténuation sonore 40 reliée au circuit d'air 15 par un troisième canal de liaison 41. Ces chambres d'atténuation sonore 20, 30, 40 constituent des résonateurs de Helmholtz, comme expliqué ci-après.

Dans ce mode de réalisation, le circuit d'air 15 du caisson 10 est tortueux, c'est-à-dire non-rectiligne. Ainsi, il présente plusieurs portions de circuit successives 15.1-15.3, comprenant une portion amont 15.1 sensiblement linéaire comprenant l'entrée de gaz 13 ; une portion aval 15.3, également sensiblement linéaire, reliée à ou débouchant dans le compartiment interne 11 logeant la turbine 12 ; et une portion de jonction 15.2 reliant fluidiquement lesdites portions amont et aval 15.1, 15.3 de manière à assurer une continuité fluidique entre ces trois portions successives 15.1-15.3.

Avantageusement, les portion amont 15.1 et aval 15.3 sont sensiblement agencées parallèles l'une à l'autre, c'est-à-dire parallèles ou approximativement parallèles. De préférence, la portion de jonction 15.2 est au moins partiellement courbe ou recourbe, c'est-à-dire qu'elle peut comprend une ou des parties sensiblement courbes/courbées et éventuellement une ou des parties non-courbes, par exemple linéaires.

Selon un mode de réalisation, la portion aval 15.3, de préférence linéaire, est reliée fluidiquement au compartiment à turbine 11 via une portion additionnelle, c'est-à-dire une portion intermédiaire aval 15.4, de préférence courbe.

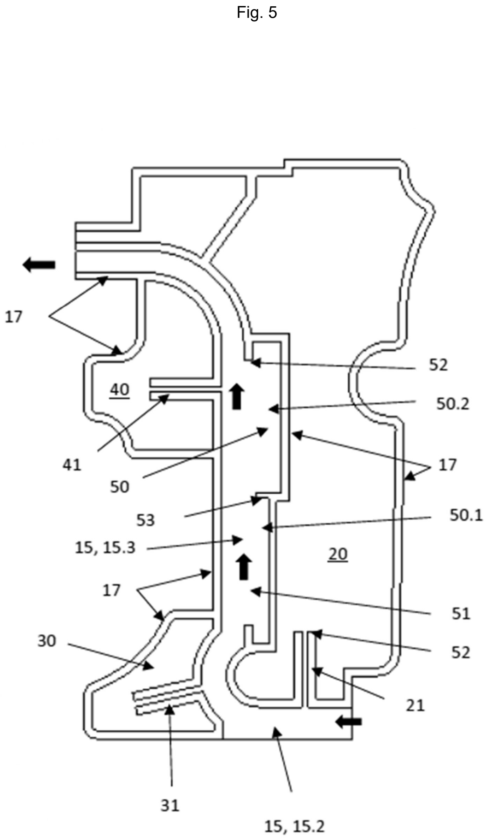

Dans le mode de réalisation des Fig. 2 à Fig. 4 et détaillé sur Fig. 5, le premier canal de liaison 21 ou premier col est en liaison fluidique avec la portion de jonction 15.2 du circuit d'air 15, et le troisième canal de liaison 41 ou troisième col est en liaison fluidique avec la portion aval 15.3 linéaire du circuit d'air 15, alors que le deuxième canal de liaison 31 ou deuxième col est en liaison fluidique avec la portion de jonction 15.2 ou la portion aval 15.3 linéaire du circuit d'air 15, en particulier il est relié au niveau de la zone de raccordement desdites portions de jonction 15.2 et aval 15.3 linéaire du circuit d'air 15.

Plus précisément, selon l'invention, la première chambre d'atténuation sonore 20 et le premier canal de liaison 21 sont dimensionnés et conformés pour constituer un premier résonateur de Helmholtz ; la deuxième chambre d'atténuation sonore 30 et le deuxième canal de liaison 31 sont dimensionnés et conformés pour constituer un deuxième résonateur de Helmholtz ; et la troisième chambre d'atténuation sonore 40 et le troisième canal de liaison 41 sont dimensionnés et conformés pour constituer un troisième résonateur de Helmholtz.

Ces résonateurs de Helmholtz constituent des atténuateurs acoustiques multifréquence. Ils sont configurés pour réduire ou atténuer le niveau sonore, c'est-à-dire le bruit généré par l'écoulement de l'air dans le circuit d'air, pendant le fonctionnement du ventilateur médical, i.e. le fonctionnement de la turbine avec aspiration d'air du fait des rotations de la roue à ailette de la turbine, et ainsi limiter la gêne sonore pour le personnel soignant et les patients, en particulier en milieu hospitalier.

Ces résonateurs de Helmholtz permettent de « pièger » les ondes sonores de différentes fréquences, comme détaillé dans le Tableau ci-après.

**Tableau**

| Résonateur | Fréquences piégées | Section du col | Longueur du col |
|---|---|---|---|
| Premier | 500 à 1000 Hz | Env. 53,5 mm² | Env. 12 mm |
| Deuxième | 400 à 900 Hz | | Env. 11 mm |
| Troisième | 1000 à 2000 Hz | | Env. 11,5 mm |

Par ailleurs, dans le mode de réalisation des Fig. 2 à Fig. 5, le circuit d'air 15 est en communication fluidique avec au moins une quatrième chambre d'atténuation sonore 50 laquelle est séparée du circuit d'air 15, i.e. de son lumen, par au moins une plaque perforée 60, c'est-à-dire une plaque qui ferme l'ouverture 51 de ladite quatrième chambre d'atténuation sonore 50 qui permet en communication fluidique la quatrième chambre d'atténuation sonore 50 et le circuit d'air 15.

Ici, la quatrième chambre d'atténuation sonore 50 est « fermée » par deux plaques perforées 60 juxtaposées, de préférence l'une des plaques perforées 60 présente une flexibilité supérieure à l'autre.

Comme détaillé sur Fig. 5, la quatrième chambre d'atténuation sonore 50 est ici en communication fluidique avec la portion aval 15.3 linéaire du circuit d'air 15. Elle est sous-divisée en deux compartiments 50.1, 50.2 d'atténuation sonore juxtaposés, séparés l'un de l'autre par une portion de paroi intermédiaire 53, c'est-à-dire une paroi intercalaire.

Chaque compartiment d'atténuation sonore 50.1, 50.2 permet d'absorber des fréquences sonores (i.e. ondes sonores) spécifiques, de préférences au moins en partie différentes d'un compartiment à l'autre, comprises entre 800 et 1500 Hz. On peut utiliser une plaque perforée 60 unique pour fermer les deux compartiments d'atténuation sonore 50.1, 50.2 ou, selon le cas, deux plaques perforées 60 fermant chacune l'un des compartiments 50.1, 50.2.

Par ailleurs, la (ou les) plaque perforée 60 est montée, de préférence de manière extractible, face à l'ouverture 51 de la quatrième chambre d'atténuation sonore 50 en étant retenue par des éléments de parois 52, 53.

Selon un mode de réalisation, la ou chaque plaque perforée a une épaisseur comprise entre 0,1 et 2 mm, de préférence entre 0,5 et 1 mm, et/ou est formée d'un matériau de type polymère, de préférence de type ABS/PC.

La ou chaque plaque perforée 60 a un taux de perforation (i.e. aire perforée/aire totale) compris entre 1 et 2%, de préférence moins de 1,5%. Elle est traversée par des trous pouvant former un quadrillage. Les trous 61, i.e. passages traversant, peuvent avoir un diamètre compris entre 0,3 et 0,8 mm.

La quatrième chambre d'atténuation sonore 50 et la (ou les) plaque perforée 60 participent également à l'atténuation sonore en « piégeant » des ondes sonores du flux d'air ayant des fréquences comprises entre 800 et 1500 Hz.

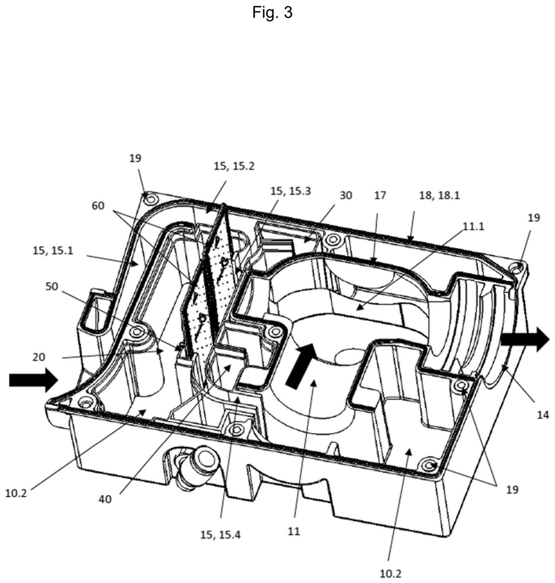

Selon un mode de réalisation préféré, les parois internes 17, notamment celles illustrées en Fig. 5, en particulier les chambres d'atténuation sonore 20-50, les canaux 21-41 et tout ou partie du circuit d'air 15, sont formées d'une pièce avec les parois du caisson, en particulier les parois de fond 10.2 des deux demi-coques 10A, 10B, comme illustré sur Fig. 3, par exemple par moulage par injection.

Toutefois, selon un autre mode de réalisation, au moins une partie desdites parois internes 17, notamment celles illustrées en Fig. 5, sont agencées sur ou dans une ou des structures amovibles/extractibles venant se loger dans le caisson 10, c'est-à-dire sur les parois de fond 10.2 des deux demi-coques 10A, 10B du caisson 10.

Par ailleurs, est prévu aussi, dans le caisson 10, une entrée d'oxygène 16 en communication fluidique avec le compartiment à turbine 11 afin de pouvoir amener de l'oxygène additionnel et réaliser un enrichissement de l'air provenant du circuit d'air 15 avec de l'oxygène. Un connecteur 70 permet de raccorder fluidique une ligne d'amenée d'oxygène à l'entrée d'oxygène 16.

En fonctionnement, l'air ambiant est aspiré par la turbine 12, pendant son fonctionnement, i.e. lorsque l'axe-moteur entraîne la roue en rotation, via l'entrée d'air 13 en communication fluidique avec l'atmosphère ambiante et le circuit d'air 15.

De préférence, l'air est filtré (non montré) avant de pénétrer dans le caisson 10, par des moyens de filtration, à savoir un filtre ou analogue, par exemple de type HEPA. Après filtration, l'air est débarrassé des polluants qu'il peut contenir, tels que poussières, microorganismes (bactéries, virus, spores..) ou autres aérosols.

La turbine motorisée 12 a une architecture classique. Elle comprend classiquement un moteur électrique agencé dans le carter 12.2. Le moteur est alimenté électriquement par des moyens d'alimentation électrique, en particulier des câbles électriques 12.4 et un connecteur 12.5 de raccordement à la carte électronique des moyens de pilotage.

Le moteur, lorsqu'il est commandé/contrôlé par les moyens de pilotage, entraine en rotation, pendant son fonctionnement, l'axe ou arbre-moteur qui porte une roue à ailettes. La roue à ailettes est agencée mobile en rotation dans le compartiment interne de la volute 12.1, par exemple prise en sandwich entre une demi-volte inférieure et une demi-volte supérieure fixée de manière étanche l'une à l'autre et délimitant entre-elles le compartiment interne de la turbine 12.

L'air aspiré par la roue à ailettes pénètre dans la volute 12.1 par une entrée de volute 12.3, i.e. une ouverture ou orifice d'entrée, située en haut de volute 12.1 et en ressort par une sortie de volute, i.e. une ouverture ou orifice de sortie, typiquement par un conduit d'évacuation d'air 12.6, en communication fluidique avec la sortie de gaz 14 du caisson 10. Ici, l'entrée de volute 12.3 se situe au-dessus de la volute 12.2 et la sortie de volute est située latéralement (i.e. conduit 12.6) mais, bien entendu, d'autres configurations sont possibles, en fonction de la turbine 12 utilisée.

Le flux gazeux sortant de la turbine 12, via le conduit 12.6, est ensuite envoyé vers un patient, via un circuit patient (non montré) alimentant une interface respiratoire, tel un masque respiratoire ou analogue (non montré). Le circuit patient, par exemple un ou plusieurs conduits flexibles, vient se raccorder fluidiquement de manière détachable, à une sortie de gaz du ventilateur 1 en liaison fluidique avec la sortie 14 du caisson 10.

Comme déjà précisé, l'air peut être enrichi en oxygène, en amont de la turbine 12, par adjonction d'oxygène au flux d'air et mélange de ces gaz, en particulier au sein du compartiment à turbine 11 ou immédiatement avant leur entrée dans la volute 12.1.

Le moteur de la turbine 12 est alimenté en courant électrique provenant du secteur (110/220V) et/ou d'une batterie rechargeable interne, par exemple via la carte électronique faisant partie des moyens de pilotage.

La vitesse de rotation maximale atteinte pendant le fonctionnement de la turbine 12 est d'au moins 25 000 tours/minute, de préférence au moins 40 tr/min, voire plus, typiquement entre 50 000 et 70 000 tr/min.

Les accélérations et/ou les décélérations/freinages du moteur sont contrôlés par lesdits moyens de pilotage, typiquement en fonction des phases inspiratoires et expiratoires du patient.

D'une façon générale, les moyens de pilotage constituent un dispositif ou une unité de commande électronique ou analogue. Ils comprennent typiquement une (ou des) carte électronique à un ou plusieurs microprocesseur(s), et pilotent la turbine 12 de manière à fournir le gaz respiratoire aux débit et pression désirés, de préférence sélectionnés ou réglés par l'utilisateur via une interface homme-machine (IHM) du ventilateur 1, par exemple un écran tactile 3 du ventilateur 1 et/ou des touches ou boutons de sélection ou analogue.

Le ou les microprocesseurs sont agencés sur la (ou les) carte électronique, avantageusement un (des) microcontrôleur, et mettent en oeuvre un ou des algorithmes, permettant notamment de monitorer et réguler la ventilation délivrée au patient en exploitant notamment des signaux de pression et de débit mesurés par des capteurs de pression et/ou de débit agencés dans le ventilateur 1 et/ou sur le circuit patient. Les mesures (i.e. signaux) de pression et de débit opérées par les capteurs sont transmises par les capteurs aux moyens de pilotage du ventilateur 1.

La (ou les) carte électronique peut aussi comprendre des moyens de mémorisation pour mémoriser notamment les valeurs de pression et/ou de débit, par exemple une mémoire type flash ou autre portée par la carte électronique, ou dans tout autre moyen de mémorisation, y compris dans un algorithme du microprocesseur.

Avantageusement, comme illustré en Fig. 1, le ventilateur 1 comprend aussi une interface homme-machine ou IHM servant d'interface de réglage ou de sélection pour l'utilisateur, i.e. le personnel soignant. L'IHM comprend ici un écran d'affichage 3 intégré à la carcasse 2 du ventilateur 1 et un bouton-rotatif 4 permettant de réaliser des choix, des sélections, des validations ou autres.

L'écran d'affichage 3 est préférentiellement un écran tactile, et avantageusement à affichage en couleurs, sur lequel s'affichent, pendant le fonctionnement du ventilateur 1, des touches virtuelles de sélection ou de réglage, actionnables par appui digitale de l'utilisateur, c'est-à-dire lorsque l'utilisateur exerce une pression/un appui dessus avec son index par exemple. Les touches virtuelles peuvent se présenter sous forme d'icônes, de fenêtres ou analogues.

Le (ou les) bouton rotatif ou autre sert par exemple à faire varier les valeurs à régler et à valider volontairement les configurations choisies, ou à d'autres fonctions.

Les moyens d'alimentation électrique du ventilateur 1 comprennent une source de courant électrique, typiquement une batterie rechargeable, un ou des câbles avec prise de raccordement au secteur (110/220V) et convertisseur de courant/tension, lesquels servent à alimenter en énergie électrique, les éléments en ayant besoin pour fonctionner, en particulier la turbine 12, les moyens de pilotage, notamment la carte(s) électronique(s) et le(s) microprocesseur(s), les capteurs de pression et/ou de débit, l'écran d'affichage 3, et/ou d'autres composants.

Bien entendu, le ventilateur 1 peut comprendre aussi d'autres éléments de fonctionnement, notamment des connecteurs servant à différents raccordements de type prise USB double, connecteur ADMI, connecteurs RS235 SUB D5 et RJ45, ou autres, un haut-parleur...

Comme déjà expliqué et illustré sur Fig. 2 à Fig. 5, selon l'invention, afin de réduire les nuisances sonores, c'est-à-dire le bruit, engendré par l'aspiration d'air par la turbine 12, lorsque la roue est mise en rotation, c'est-à-dire pendant son fonctionnement, on agence dans le circuit d'air 15, plusieurs des résonateurs de Helmoltz, à savoir les chambres d'atténuation sonore 20, 30, 40 associées à des canaux de liaison 21, 31, 41 par lesquels les ondes sonores véhiculées avec l'air circulant dans le circuit d'air 15, peuvent pénétrer dans les chambres d'atténuation sonore 20, 30, 40 et y être atténuées, c'est-à-dire y être piégées et absorbées.

Comme détaillé ci-avant, les résonateurs de Helmholtz sont agencés dans le circuit d'air 300, en amont de l'entrée du compartiment 11 logeant la turbine 12, en considérant le sens de circulation de l'air aspiré allant de l'entrée d'air 13 vers la turbine 12 (cf. Flèches noires sur Fig. 3).

Afin de garantir une bonne efficacité dans l'atténuation des ondes sonores audibles nuisibles, c'est-à-dire du bruit résultant de l'aspiration et de la circulation de l'air, notamment dans le circuit d'air 15, les dimensions et les configurations des résonateurs sont choisis avec soin, comme détaillé dans le Tableau ci-avant.

Les dimensions et configurations des résonateurs peuvent être déterminées par de simples essais empiriques ou par simulation car peuvent dépendre de la géométrie de l'ensemble du caisson 10.

Les chambres d'atténuation sonore 20, 30, 40 des résonateurs forment chacune une cavité de volume interne donné, typiquement compris entre 10 et 90 cm³.

Les dimensions des canaux de jonction 21, 31, 41 ou cols sont typiquement une section intérieure à 70 mm² et une longueur inférieure à 15 mm, par exemple les dimensions donné dans le Tableau ci-avant.

Ces canaux de jonction 21, 31, 41 ou cols viennent se raccorder fluidiquement au circuit d'air 15, en particulier dans ses portions 15.2, 15.3 comme déjà expliqué afin de piéger/atténuer les ondes sonores nuisibles. Avantageusement, les canaux de jonction 21, 31, 41 se projettent, c'est-à-dire font saillie, vers l'intérieur des chambres d'atténuation sonore 20, 30, 40 des résonateurs, comme mieux visible en Fig. 5.

La (les) plaque perforée 60 et la quatrième chambre 50 participent également au piégeage des ondes sonores.

Des essais réalisés avec un ventilateur 1 équipé d'un caisson selon l'invention ont montré une forte limitation/atténuation des sifflements, ronronnements et/ou autres bruits sourds audibles générés par la circulation/écoulement de l'air dans le circuit interne, pendant le fonctionnement de la turbine 12, typiquement jusqu'à 20 ou 30 dBA environ en fonction des fréquences des ondes sonores piégées, c'est-à-dire selon le résonateurs de Helmholz 20, 21 ; 30, 31 ; 40, 41 et/ou la quatrième chambre 50 considérés.

En d'autres termes, les trois résonateurs de Helmholz 20, 21 ; 30, 31 ; 40, 41 et la plaque perforée 60 associée à la quatrième chambre 50 permettent de piéger des ondes sonores, de même fréquence et/ou de fréquences différentes, comprises dans une plage large de fréquences, à savoir entre 400 et 2000 Hz, et ainsi diminuer le bruit généré lors du fonctionnement de la turbine 12 du fait de l'écoulement du flux d'air dans le circuit d'air 15, et ainsi minimiser ou réduire la gêne pour les utilisateurs : patients, médecins ou autres.

Un avantage majeur du caisson 10 de l'invention est qu'elle permet d'éviter toute utilisation de mousses d'insonorisation.

D'une façon générale, le ventilateur médical 1 de l'invention est particulièrement bien adapté à une utilisation en milieu hospitalier mais peut être aussi utilisé lors des interventions d'urgence sur le terrain de type SAMU, ambulance, pompiers....

## Revendications

1. Ventilateur médical (1) comprenant : un caisson à turbine (10) comprenant un compartiment interne (11) logeant une turbine motorisée (12), et un circuit d'air (15) reliant fluidiquement une entrée d'air (13) au compartiment à turbine (11), et des moyens de pilotage pilotant la turbine motorisée (12), **caractérisé en ce que** le circuit d'air (15) est en communication fluidique avec :
- une première chambre d'atténuation sonore (20) et un premier canal de liaison (21) dimensionnés et conformés pour former un premier résonateur de Helmholtz configuré pour piéger, stopper et/ou atténuer des ondes sonores de fréquence comprise entre 500 et 1000 Hz,
- une deuxième chambre d'atténuation sonore (30) et un deuxième canal de liaison (31) dimensionnés et conformés pour former un deuxième résonateur de Helmholtz configuré pour piéger, stopper et/ou atténuer des ondes sonores de fréquence comprise entre 400 et 900 Hz,
- une troisième chambre d'atténuation sonore (40) et un troisième canal de liaison (41) dimensionnés et conformés pour former un troisième résonateur de Helmholtz configuré pour piéger, stopper et/ou atténuer des ondes sonores de fréquence comprise entre 1000 et 2000 Hz,
- et au moins une quatrième chambre d'atténuation sonore (50), via au moins une plaque perforée (60), la quatrième chambre d'atténuation sonore (50) et ladite au moins une plaque perforée (60) étant configurées pour piéger, stopper et/ou atténuer des ondes sonores de fréquence comprise entre 800 et 1500 Hz.

2. Ventilateur selon la revendication 1, **caractérisé en ce que** le caisson (10) a une forme parallélépipédique rectangle et/ou comprend une entrée de gaz (13) et une sortie de gaz (14) agencés dans la paroi périphérique (10.1 ; 10.11, 10.12) du caisson (10).

3. Ventilateur selon l'une des revendications 1 ou 2, **caractérisé en ce que** le caisson (10) comprend deux demi-coques (10A, 10B) solidarisées l'une à l'autre, de préférence un joint d'étanchéité est agencé entre les deux demi-coques (10A, 10B).

4. Ventilateur selon l'une des revendications 1 à 3, **caractérisé en ce que** le circuit d'air (15) du caisson (10) est tortueux.

5. Ventilateur selon l'une des revendications 1 à 4, **caractérisé en ce que** le circuit d'air (15) comprend :
- une portion amont (15.1) linéaire comprenant l'entrée de gaz (13),
- une portion aval (15.3) linéaire débouchant dans le compartiment interne (11) logeant la turbine (12), et
- une portion de jonction (15.2) reliant fluidiquement lesdites portions amont et aval (15.1, 15.3), de préférence une portion de jonction (15.2) au moins partiellement courbe.

6. Ventilateur selon les revendications 1 et 5, **caractérisé en ce que** :
- le premier canal de liaison (21) est en liaison fluidique avec la portion de jonction (15.2) du circuit d'air (15),
- le troisième canal de liaison (41) est en liaison fluidique avec la portion aval (15.3) linéaire du circuit d'air (15), et
- le deuxième canal de liaison (31) est en liaison fluidique avec la portion de jonction (15.2) et/ou la portion aval (15.3) linéaire du circuit d'air (15), en particulier le deuxième canal de liaison (31) est relié à la jonction desdites portion de jonction (15.2) et portion aval (15.3) linéaire du circuit d'air (15).

7. Ventilateur selon la revendication 1, **caractérisé en ce que** la quatrième chambre d'atténuation sonore (50) est en communication fluidique avec la portion aval (15.3) linéaire du circuit d'air (15).

8. Ventilateur selon la revendication 1, **caractérisé en ce que** la ou chaque plaque perforée est traversée par des trous ayant un diamètre compris entre 0,3 et 0,8 mm.

9. Ventilateur selon la revendication 1, **caractérisé en ce que** ladite au moins une plaque perforée (60) a un taux de perforation compris entre 1 et 2%.

10. Ventilateur selon l'une des revendications précédentes, **caractérisé en ce qu'**au moins le compartiment interne (11) logeant la turbine motorisée (12), le circuit d'air (15), les chambres d'atténuation sonore (20, 30, 40) et les canaux de liaison (21, 31, 41) sont délimités par des parois internes (17) agencées dans le caisson (10).

11. Ventilateur selon la revendication 1, **caractérisé en ce que** la première chambre d'atténuation sonore (20) a un volume différent de celui des deuxième et troisième chambres d'atténuation sonore (30, 40).

12. Ventilateur selon la revendication 11, **caractérisé en ce que** les première, deuxième et troisième chambres d'atténuation sonore (20, 30, 40) ont un volume interne compris entre 10 et 90 cm³.

13. Ventilateur selon l'une des revendications 1 ou 8, **caractérisé en ce que** la ou chaque plaque perforée a une épaisseur comprise entre 0,1 et 2 mm.

14. Ventilateur selon la revendication 1, **caractérisé en ce que** la quatrième chambre d'atténuation sonore (50) est sous-divisée en deux compartiments d'atténuation sonore (50.1, 50.2) séparés par une paroi intercalaire (53).

15. Ventilateur selon la revendication 1, **caractérisé en ce que** les premier, deuxième et troisième canaux de liaison se projettent vers l'intérieur des première, deuxième et troisième chambres d'atténuation sonore
